Europäisches Patentamt

European Patent Office

Office européen des brevets

(11) Veröffentlichungsnummer: **0 203 463 B1**

# EUROPÄISCHE PATENTSCHRIFT

(45) Veröffentlichungstag der Patentschrift: 06.11.91

(51) Int. Cl.5: **G01N 30/48, B01J 20/32**

(21) Anmeldenummer: 86106574.6

(22) Anmeldetag: 14.05.86

Die Akte enthält technische Angaben, die nach
dem Eingang der Anmeldung eingereicht wurden
und die nicht in dieser Patentschrift enthalten sind.

(54) **Verfahren zur Herstellung eines Materials zur Affinitätschromatographie.**

(30) Priorität: 25.05.85 DE 3519011

(43) Veröffentlichungstag der Anmeldung:
03.12.86 Patentblatt 86/49

(45) Bekanntmachung des Hinweises auf die
Patenterteilung:
06.11.91 Patentblatt 91/45

(84) Benannte Vertragsstaaten:
AT BE CH DE FR GB IT LI LU NL SE

(56) Entgegenhaltungen:
EP-A- 0 094 892
EP-A- 0 110 409
FR-A- 2 403 098

(73) Patentinhaber: **BEHRINGWERKE Aktiengesellschaft
Postfach 1140
W-3550 Marburg 1(DE)**

(72) Erfinder: **Stüber, Werner, Dr.
Cölber Weg 12
W-3551 Lahntal(DE)**
Erfinder: **Pâques, Eric-Paul, Dr.
Schmiedeacker 18
W-3550 Marburg(DE)**

(74) Vertreter: **Klein, Otto, Dr. et al
Hoechst AG Zentrale Patentabteilung Postfach 80 03 20
W-6230 Frankfurt am Main 80(DE)**

**Beschreibung**

Die Erfindung betrifft ein Verfahren zur Herstellung eines Materials zur Affinitätschromatographie, worin ein oxidiertes sulfatiertes Polysaccharid an ein aminogruppentragendes Trägermaterial gebunden ist.

Dieses Material kann zur Isolierung und Reinigung von Stoffen gebraucht werden, die mit sulfatierten Polysacchariden in Wechselwirkung treten.

Einleitung

In den letzten Jahren wurde die Isolierung von Enzymen durch die Technik der Affinitätschromatographie wesentlich verbessert. Diese Methode nutzt spezifische Wechselwirkungen zwischen Substanzen aus. Dazu wird eine Substanz als Ligand an einen unlöslichen Träger (Matrix) kovalent gebunden. Der Ligand muß mit der zu isolierenden Substanz in eine komplexartige Wechselwirkung treten können. Der Ligand hält nur die Substanzen zurück, die spezifisch mit ihm reagieren. Andere Stoffe werden ausgewaschen. Die zurückgehaltenen Substanzen können vom Trägermaterial mittels einer Lösung von ungebundenem Liganden oder beispielsweise mit einem Salzgradienten eluiert werden.

Der Erfolg einer Affinitätschromatographie hängt davon ab, wie gut die natürlicherweise zwischen Ligand und zu isolierender Substanz vorhandenen Wechselwirkungen nachgeahmt werden. Wichtig ist deshalb die Wahl der Matrix und die Art der Ligandenimmobilisierung. Die Matrix sollte hydrophil sein und gute mechanische und chemische Stabilitäten besitzen. Die Wechselwirkung hindernde sterische Effekte können durch Spacer günstig beeinflußt werden. Weder die Matrix noch der Spacer sollten zu einer unspezifischen Adsorption Anlaß geben.

Zur Isolierung eines Proteins ist es natürlich am günstigsten, einen Liganden zu benutzen, der nur mit einem oder wenigen Proteinen Wechselwirkungen eingeht. Die Kapazität des Adsorbens ist von einer genügend hohen Ligandenbeladung der Matrix abhängig. Die chemische Bindung sollte möglichst einheitlich und stabil, das heißt schwer hdrolysierbar sein.

Die Affinitätschromatographie läßt sich zur Isolierung von Proteinen, beispielsweise aus Plasma einsetzen, besonders von Antithrombin III. Als Affinitätsmaterial haben sich dabei immobilisierte, sulfatierte Polysaccharide, besonders trägergebundenes Heparin, bewährt. Es ist jedoch bekannt, daß bei der Immobilisierung von Heparin und anderen sulfatierten Polysacchariden Modifikationen des Liganden mit geringerer biologischer Aktivität entstehen können.

Der vorliegenden Erfindung lag die Aufgabe zugrunde, ein Material zur Affinitätschromatographie herzustellen, indem ein sulfatiertes Polysaccharid kovalent an einen Träger gebunden wird, so daß ein Material mit großer biologischer Aktivität, Ligandendichte und Stabilität erhalten wird.

Stand der Technik

Zur Isolierung von Proteinen, die mit sulfatierten Polysacchariden Komplexe bilden, wird die Affinitätschromatographie besonders unter Verwendung von Heparin benutzt.

Dazu wird ein sulfatiertes Polysaccharid an einen geeigneten Träger gebunden, wobei häufig das Mucopolysaccharid Heparin das Polysaccharid darstellt. Als Trägermatrix hat sich besonders Sepharose® 4B (ein Agarose-Gel in Perlform; Pharmacia, Schweden) bewährt.

Die kovalente Verbindung eines sulfatierten Polysaccharids mit dem Träger wird vornehmlich durch eine Aktivierung des Trägermaterials oder des sulfatierten Polysaccharids mit Bromcyan erreicht (Thromb.Res. 5, 439-452 (1974), deutsche Offenlegungsschrift 22 43 688). Diese Methode ist jedoch mit Mängeln behaftet. Die sich zwischen Aminogruppen von Liganden und Hydroxygruppen üblicher Trägermaterialien ausbildenden Isoharnstoffbindungen weisen gegenüber nucleophilen Reagenzien eine gewisse Labilität auf. Das bedeutet, daß unter Elutionsbedingungen, insbesondere bei erhöhtem pH-Wert, mit einer Ligandenablösung zu rechnen ist (Enzyme Microb. Technol. 4, 161-163, 1981).

Dieser Nachteil kann durch den Ersatz des Bromcyans durch oxirangruppenhaltige Aktivierungsstoffe behoben werden. Oxirangruppen lassen sich in eine hydroxygruppenenthaltende Matrix mit Epichlorhydrin (J. Chromatogr. 51, 479, 1970) oder mit Bis-oxiranen wie 1,4-Butandiolbis-(epoxypropylether) (J.Chromatogr. 90, 87, 1974) einführen. Die eingeführten Oxirangruppen können mit Ammoniak in Aminogruppen übergeführt werden. An diese Aminogruppen läßt sich ein carboxylgruppen-enthaltendes, sulfatiertes Polysaccharid mittels eines Carbodiimids binden (Anal.Biochem. 126, 414-421, 1982).

Die resultierenden Amidbindungen zeichnen sich durch eine hohe chemische Stabilität aus. Der Nachteil dieser Methode liegt darin, daß während der Aktivierung mit Carbodiimiden eine Anzahl von Carboxylgruppen in N-Acylharnstoffderivate umgewandelt werden. Das bedeutet, daß nach diesem Verfahren zwar eine hohe Beladung des Trägers mit sulfatierten Polysacchariden erreicht wird, infolge ihrer chemischen Modifikation aber vergleichsweise niedere Bindungskapazitäten für die mittels Affinitätschromatographie zu isolierenden Stoffe vorliegen.

Die Verbindung eines sulfatierten Polysaccharids mit einem aminofunktionalisierten Trägermaterial kann jedoch in chemisch eindeutiger Weise durch eine reduktive Kupplung erreicht werden (Analyt.Biochem. 126, 414-421, 1982). Hierbei wird das reduzierende Ende (Aldehydgruppe) der Saccharidkette an eine Aminogruppe eines Polymers unter Ausbildung einer Schiffschen Base gekuppelt. Zur Stabilisierung der C = N-Doppelbindungen werden diese beispielsweise mit Natriumcyanborhydrid zum sekundären Amin reduziert. Diese Methode gestattet eine hinreichende Beladung von aminofunktionalisierter Sepharose[R] 4B mit beispielsweise Heparin, ist jedoch auf andere Polymere nicht problemlos übertragbar.

Anstelle der vielfach eingesetzten Sepharose 4B können prinzipiell andere hydroxylgruppen-haltige Polymere als Trägermaterial benutzt werden, beispielsweise Fractogel[R] HW-65 (F) (ein synthetisches hydrophiles hydroxylgruppen-haltiges Polymer; J.Chromatogr. 239, 747-754, 1982). Dieses Polymer ist aufgrund seiner chemischen und physikalischen Eigenschaften für die großtechnische Anwendung oft günstiger als Sepharose[R] 4B.

In EP-A-110 409 sind polymergebundene sulfatierte Polysaccharide beschrieben, insbesondere polymergebundenes Dextransulfat, die für den extrakorporalen Kreislauf zur Entfernung von Lipoproteinen, Viren und gefährlichen Zellen geeignet sind. Die darin beschriebenen Materialien sind jedoch zur Gewinnung von Proteinen, die in der Blutgerinnung eine Rolle spielen, wenig geeignet, da sie insbesondere hochlipophile Eigenschaften Zeigen und zudem eine zu geringe Kapazität gegenüber wichtigen Proteinen wie AT III zeigen. Die Anreicherung von Lipoproteinen stellt in diesem Zusammenhang eine unerwünschte Eigenschaft dar. Es werden keine Materialien zur Verfügung gestellt, mit denen solche Proteine in einem großen Maßstab, mit hohen Ausbeuten, guter Reinheit, mit hoher Geschwindigkeit und ohne Ligandenverlust des Materials beim wiederholten Gebrauch gewonnen werden können.

Es ist jedoch kein Verfahren bekannt, [R]Fractogel HW-65 (F) nach Aminofunktionalisierung in befriedigender Ausbeute an das reduzierende Ende eines sulfatierten Polysaccharids zu binden.

Das in dieser Anmeldung beschriebene Verfahren weist die Nachteile des Standes der Technik nicht auf und macht es möglich, ein geeignet derivatisiertes, sulfatiertes Polysaccharid mit guten Ausbeuten an ein aminofunktionalisiertes Trägermaterial zu kuppeln.

Gegenstand der Erfindung

Im beschriebenen Verfahren werden sulfatierte Polysaccharide mit einem diolspaltenden Oxidationsmittel modifiziert, wobei zusätzliche Aldehydgruppen am Polysaccharid geschaffen werden. Derartige Derivate lassen sich in guten Ausbeuten an aminofunktionalisierte Träger binden. Die entstandenen Schiffschen Basen können mit Reduktionsmitteln, zum Beispiel Natriumcyanborhydrid, zu Amin reduziert werden.

Gegenstand der Erfindung ist deshalb ein Material zur Affinitätschromatographie erhältlich durch Reaktion eines Aminogruppen tragenden Copolymeren aus Methacrylsäurederivaten, Pentaerythrit, Polyethylenglykol und Divinylbenzol mit einem sulfatierten Polysaccharid, das mit einem Oxidationsmittel behandelt wurde, das Glykole unter Spaltung der Kohlenstoffkette zu Aldehyden oxidiert, und Behandlung des entstandenen Produkts mit einem Reduktionsmittel, dadurch gekennzeichnet, daß als sulfatiertes Polysaccharid Heparin verwendet wird.

Als Ausgangsmaterial wird Heparin oder dessen Natriumsalz eingesetzt. Aldehydgruppen wurden in wäßriger Lösung durch Behandeln mit einem zur Reaktion mit Diolen unter Bildung von Aldehydgruppen bekannten Oxidationsmittel, vorzugsweise einem Alkaliperjodat, erzeugt. Der pH-Wert der Reaktionslösung wurde mit einer Base, vorzugsweise einem Alkalihydroxid, besonders Lithiumhydroxid, im Bereich von 5-9, vorzugsweise 6-8, gehalten. 5-100 mg Alkaliperjodat, bevorzugt 30-40 mg Natriumperjodat, pro Gramm Heparin erwiesen sich als besonders günstig. Die Reaktionszeiten liegen im Bereich von 10 Min. bis 5 Std., wobei die Reaktionstemperatur zwischen 0 und 30 °C gehalten wird. Bevorzugt wurde die Oxidationsreaktion während einer Stunde bei 4 °C ausgeführt.

Zur Kupplung des polyaldehydderivatisierten sulfatierten Polysaccharids kann der Oxidationsansatz direkt zu einem aminofunktionalisierten Träger gegeben werden. Aminofunktionalisierte Träger sind in Analyt.Biochem. 126, 414-421 (1982) beschrieben.

Als zu funktionalisierendes Trägermaterial wird ein hydroxylgruppen-haltiges unlösliches Polymer, das sich in geeigneter Weise aminofunktionalisieren lässt nämlich ein Copolymerisat aus Methacrylsäurederivaten, Pentaerythrit, Polyethylenglykol und Divinylbenzol, wie sie unter der Bezeichnung [R]Fractogel vertrieben werden, benutzt.

Ein auf diese Weise hergestellter aminofunktionalisierter Träger wird mit dem polyaldehydderivatisierten Polysaccharid umgesetzt, vorzugsweise 30-40 g Polysaccharid mit 1000 ml Träger. Die Reaktion wird bei pH 6-9, bevorzugt bei Raumtemperatur, ausgeführt, vorzugsweise in einer gepufferten wäßrigen Lösung, insbesondere bei pH 6-7 in einem Phosphatpuffer. Nach Vereinigung der Reaktanten wird Natriumcyanborhydrid zugesetzt. Bei Raumtemperatur dauert die Umsetzung 1 bis 30 Tage. Die bevorzugte Reaktionszeit ist 12 bis 16 Tage. Das Produkt wird mit Wasser gewaschen und mit Acetanhydrid behandelt, wodurch noch freie Aminogruppen acetyliert werden.

Es ist bekannt, daß Diole mit Hydroxygruppen an benachbarten Kohlenstoffatomen (Glykole) wie beispielsweise Zucker mit geeigneten Oxidationsmitteln unter Spaltung der Kohlenstoff-Kohlenstoff-Bindung Aldehydgruppen liefern. Überraschenderweise führte die Einwirkung der nötigen "starken" Oxydationsmittel nicht zu einem Verlust an biologischer Aktivität der sulfatierten Polysaccharide und nach dem beschriebenen Verfahren polyaldehydderivatisiertes Heparin zeigte eine hohe Bindungsaffinität und Aktivität gegenüber Proteinen, die Komplexe mit Heparin bilden. Diese biologische Aktivität bleibt auch in den trägergebundenen Derivaten erhalten. Durch die hohe Anzahl von Aldehydgruppen in den Liganden wird ein günstiges Kupplungsverhalten erreicht, denn die Kupplungsausbeuten hängen sowohl von der Zahl der Aminogruppen am Träger als auch von der Zahl der Aldehydgruppen am Liganden ab. Träger mit wenigen Aminogruppen bedürfen einer hohen Anzahl reaktiver Aldehydgruppen. Die Bindungskapazität des Adsorbens für das zu adsorbierende Protein ist direkt abhängig von der Lingandenbeladung.

Mit den auf die beschriebene Weise erhaltenen Affinitätsmaterialien können Proteine, die an sulfatierte Polysaccharide binden, aus Lösungen dieser Proteine adsorbiert und gegebenenfalls dann davon desorbiert werden, beispielsweise Antithrombin III aus Blutplasma. Der Einfluß der Aminozahl der Harze auf die Kupplungsausbeuten von Heparin und polyaldehydderivatisiertem Heparin wurde mit den bevorzugt eingesetzten Polymeren Sepharose® 4B und Fractogel® HW-65 (F) nachgewiesen.

Wurde ein nach dieser Methode polyaldehydderivatisiertes Heparin an Aminofractogel HW-65(F) gebunden, stieg die Bindungkapazität auf den 2- bis 5-fachen Wert im Vergleich zum unverändert immobilisierten Liganden.

Aufgrund der chemischen Natur der Ligandenverknüpfung zeigten die Adsorbentien, die mit Hilfe der derivatisierten (oxydierten), sulfatierten Polysaccharide hergestellt wurden, eine sehr geringe Tendenz zum Ligandenverlust. Im Vergleich zu einem über Isoharnstoffbindungen, wie sie durch Bromcyanaktivierung entstehen, immobilisierten Liganden wurde der Ligandenverlust durch das beschriebene Verfahren auf 0,1 bis 1 % unter vergleichbaren Bedingungen zurückgedrängt.

Beispiel 1

Oxidation von Heparin-Natrium-Salz

30 g Heparin-Natrium-Salz (etwa 160 IE/mg) wurden in 500 ml Wasser gelöst und mit einer 20 g/l Lithiumhydroxidlösung auf pH 7 gebracht. Diese Lösung wurde auf 4°C gekühlt und mit 1,2 g Natriumperjodat versetzt. Die Oxidation wurde während 1 Stunde durchgeführt, wobei der pH-Wert durch Zutropfen einer 20 g/l Lithiumhydroxidlösung auf 7 gehalten wurde. Diese Lösung wurde direkt zur Kupplung an den aminofunktionalisierten Träger benutzt.

Beispiel 2

Aminofunktionalisierung von Fractogel^R HW-65 (F)

1000 ml Fractogel^R HW-65 (F) wurden mit 325 ml Wasser und 275 ml 5 normaler Natronlauge versetzt. Hierzu gab man 200 ml Epichlorhydrin und schüttelte den Ansatz 2 Stunden bei 45°C. Das Produkt wurde abfiltriert und mehrmals mit Wasser gewaschen. Die Aminofunktionalisierung wurde bei 45°C während 90 min mit 500 ml Ammoniaklösung (Dichte 0,91 g/ml) durchgeführt. Anschließend wurde das Harz abfiltriert und bis zur Neutralreaktion mit Wasser gewaschen.

Kupplung von oxidiertem Heparin an aminofunktionalisiertes Fractogel® HW-65 (F)

1000ml Aminofractogel HW-65 (F) wurden in 500 ml 0,5 mol/l Natriumphosphatpuffer pH 6,5 suspendiert und mit 30 g nach Beispiel 1 oxidiertem Heparin versetzt. Der pH-Wert wurde mit ortho-Phosphorsäure (850 g/l Lösung) auf 6,5 gehalten. Dazu wurden 11,5 g Natriumcyanborhydrid gegeben, die in 30 ml Wasser gelöst waren. Es wurde 16 Tage bei Raumtemperatur gerührt, abfiltriert und mit Wasser waschen. Das Produkt wurde in 100 ml 0,2 mol/l Natriumacetatlösung suspendiert, mit 500 ml Acetanhydrid versetzt und 30 min bei 4°C gerührt. Es wurde abgesaugt und mit Wasser gewaschen.

**Patentansprüche**

1. Material zur Affinitätschromatographie erhältlich durch Reaktion eines Aminogruppen tragenden Copolymeren aus Methacrylsäurederivaten, Pentaerythrit, Polyethylenglykol und Divinylbenzol mit einem sulfatierten Polysaccharid, das mit einem Oxidationsmittel behandelt wurde, das Glykole unter Spaltung der Kohlenstoffkette zu Aldehyden oxidiert, und Behandlung des entstandenen Produkts mit einem Reduktionsmittel, dadurch gekennzeichnet, daß als sulfatiertes Polysaccharid Heparin verwendet wird.

2. Verfahren zur Herstellung eines Materials zur Affinitätschromatographie auf Basis eines Aminogruppen tragenden Copolymers aus Methacrylsäurederivaten, Pentaerythrit, Polyethylenglykol und Divinylbenzol, dadurch gekennzeichnet, daß Heparin mit einem Oxidationsmittel behandelt wird, das Glykole unter Spaltung der Kohlenstoffkette zu Aldehyden oxidiert, und daß dieses oxidierte Heparin mit einem solchen Copolymeren reagieren gelassen wird und das entstandene Material einer Behandlung mit einem Reduktionsmittel unterworfen wird.

3. Verfahren nach Anspruch 2, dadurch gekennzeichnet, daß Heparin in wässriger Lösung mit 5-100 mg Alkaliperjodat pro Gramm Heparin behandelt wird.

4. Verfahren nach mindestens einem der Ansprüche 2 und 3, dadurch gekennzeichnet, daß die Behandlung von Heparin in einem pH-Bereich von 6 bis 8 erfolgt.

5. Verfahren nach mindestens einem der Ansprüche 2 und 3, dadurch gekennzeichnet, daß die Oxidation von Heparin in einem Bereich von 0 bis 30° C ausgeführt wird.

6. Verfahren nach mindestens einem der Ansprüche 2 und 3, dadurch gekennzeichnet, daß die Oxidation von Heparin in einem Zeitraum von 10 Minuten bis 5 Stunden abläuft.

7. Verwendung eines Materials nach Anspruch 1 oder erhalten nach mindestens einem der Ansprüche 2 bis 6 in einem Verfahren, in welchem ein Protein, das an Heparin bindet, an dieses Material adsorbiert und gegebenenfalls davon desorbiert wird.

8. Verwendung nach Anspruch 7, dadurch gekennzeichnet, daß das Protein Antithrombin III ist.

**Claims**

1. A material for affinity chromatography obtainable by reaction of a copolymer which has amino groups and is derived from methacrylic acid derivatives, pentaerythritol, polyethylene glycol and divinylbenzene with a sulfated polysaccharide which has been treated with an oxidizing agent which oxidizes glycols to aldehydes with cleavage of the carbon chain, and treatment of the resulting product with a reducing agent, which comprises using heparin as sulfated polysaccharide.

2. A process for preparing a material for affinity chromatography based on a copolymer which has amino groups and is derived from methacrylic acid derivatives, pentaerythritol, polyethylene glycol and divinylbenzene, which comprises treating heparin with an oxidizing agent which oxidizes glycols to aldehydes with cleavage of the carbon chain, and comprises this oxidized heparin being allowed to react with a copolymer of this type, and the resulting material being subjected to a treatment with a reducing agent.

3. The process as claimed in claim 2, wherein heparin is treated in aqueous solution with 5-100 mg of alkali metal periodate per gram of heparin.

4. The process as claimed in at least one of claims 2 and 3, wherein the treatment of heparin is carried out in a pH range from 6 to 8.

5. The process as claimed in at least one of claims 2 and 3, wherein the oxidation of heparin is carried out in a range from 0 to 30° C.

6. The process as claimed in at least one of claims 2 and 3, wherein the oxidation of heparin takes place in a period of from 10 minutes to 5 hours.

7. The use of a material as claimed in claim 1 or obtained as claimed in at least one of claims 2 to 6 in a process in which a protein which binds to heparin is adsorbed onto this material and, where appropriate, desorbed therefrom.

8. The use as claimed in claim 7, wherein the protein is antithrombin III.

**Revendications**

1. Matériau pour la chromatographie d'affinité, préparable par réaction d'un copolymère de dérivés d'acide méthacrylique, pentaérythritol, polyéthylèneglycol et divinylbenzène, portant des groupes amino, avec un polysaccharide sulfaté qui a été traité par un oxydant qui oxyde les glycols en aldéhydes avec coupure de la chaîne carbonée, et traitement par un réducteur du produit résultant, caractérisé en ce que l'on utilise l'héparine en tant que polysaccharide sulfaté.

2. Procédé pour la préparation d'un matériau pour la chromatographie d'affinité, à base d'un copolymère de dérivés d'acide méthacrylique, pentaérythritol, polyéthylèneglycol et divinylbenzène, portant des groupes amino, caractérisé en ce que l'on traite de l'héparine par un oxydant qui oxyde les glycols en aldéhydes avec coupure de la chaîne carbonée, et en ce que l'on fait réagir avec un tel copolymère cette héparine oxydée, et on soumet le matériau résultant à un traitement par un réducteur.

3. Procédé selon la revendication 2, caractérisé en ce que l'on traite de l'héparine en solution aqueuse par 5-100 mg de periodate alcalin par g d'héparine.

4. Procédé selon au moins l'une des revendications 2 et 3, caractérisé en ce que le traitement de l'héparine est effectué dans une intervalle de pH de 6 à 8.

5. Procédé selon au moins l'une des revendications 2 et 3, caractérisé en ce que l'oxydation de l'héparine est effectuée dans une plage de 0 à 30° C.

6. Procédé selon au moins l'une des revendications 2 et 3, caractérisé en ce que l'oxydation de l'héparine se déroule en une durée de 10 minutes à 5 heures.

7. Utilisation d'un matériau selon la revendication 1 ou obtenu selon au moins l'une des revendications 2 à 6, dans un procédé dans lequel une protéine qui se lie à l'héparine est adsorbée sur ce matériau et éventuellement désorbée hors de celui-ci.

8. Utilisation selon la revendication 7, caractérisée en ce que la protéine est l'antithrombine III.